# EUROPEAN PATENT APPLICATION

(11) **EP 4 545 035 A1**
(43) Date of publication of application: **30.04.2025**
(21) Application number: 24208528.0
(22) Date of filing: 24.10.2024
(51) Int. Cl.: A61B 34/10, A61B 17/00, A61B 34/20, A61B 34/00, A61B 18/00, A61B 18/14

(54) **GRAPHICALLY ENCODED ABLATION TAGS ACCORDING TO REGIONS OF ANATOMY**

(30) Priority: 25.10.2023 US 202318383726
(71) Applicant: Biosense Webster (Israel) Ltd., 2066717 Yokneam (IL)
(72) Inventor: ALON, Talia, 2066717 Yokneam (IL); AZARIA, Elad, 2066717 Yokneam (IL); BREZNER, Deborah, 2066717 Yokneam (IL); BAR NOY, Eran Azriel, 2066717 Yokneam (IL); LUKHANIN, Shavit Tabo, 2066717 Yokneam (IL)
(74) Representative: Carpmaels & Ransford LLP

(57) **Abstract**

A method includes receiving at least one of locations of multiple ablation sites and coordinates of one or more anatomical regions over a surface of a heart. The at least one of ablation sites and regions are categorized according to predefined categories. The at least one of sites and anatomical regions are graphically encoded on an anatomical map according to their respective categories. The graphically encoded anatomical map is displayed to a user.

## Description

### FIELD OF THE DISCLOSURE

This disclosure relates generally to the analysis of electroanatomical (EA) maps, and specifically to a system and method for executing tagging of ablation sites, including on EA maps.

### BACKGROUND OF THE DISCLOSURE

Analysis of ablation points located over an inner wall of a cardiac chamber was previously proposed in the patent literature. For example, U.S. patent 8,900,225 describes a method for performing a medical procedure that includes bringing a probe into contact with an organ in a body of a patient. A map of the organ is displayed, and the location of the probe relative to the map is tracked. A therapy is applied via the probe at multiple tissue sites in the organ with which the probe is brought into contact. The stability of the contact between the probe and the tissue sites is assessed while applying the therapy. The map is automatically marked, responsively to the assessed stability, to indicate the tissue sites at which the therapy was applied.

As another example, U.S. patent 9,757,182 describes a method including receiving locations of multiple ablation sites formed on a surface of a heart. Distances are measured among at least some of the ablation sites based on the locations. One or more gaps between the ablation sites, which meet an alerting criterion, are identified. The identified gaps are indicated to an operator.

The present disclosure will be more fully understood from the following detailed description of the examples thereof, taken together with the drawings, in which:

### BRIEF DESCRIPTION OF THE DRAWINGS

Fig. 1 is a schematic, pictorial illustration of a catheter-based electroanatomical (EA) mapping and ablation system, in accordance with an example of the present disclosure;
Figs. 2A and 2B are EA maps superimposed with ablation sites graphically encoded according to anatomical regions, and graphically encoded according to types of ablation sources, respectively, in accordance with an example of the present disclosure;
Fig. 3 is a schematic illustration of a graphical user interface (GUI) used for categorizing and graphically encoding EA map according to anatomical regions, and user-specified categories, in accordance with an example of the present disclosure;
Figs. 4A and 4B are EA maps superimposed with ablation sites and anatomical regions graphically encoded according to anatomical regions, and according to user focus of interest, respectively, in accordance with an example of the present disclosure; and
Fig. 5 is a flow chart that schematically illustrates a method to graphically encode ablation sites and regions on an EA map, in accordance with an example of the present disclosure.

### DETAILED DESCRIPTION OF EXAMPLES

### OVERVIEW

In a typical medical ablation procedure, such as pulmonary vein isolation (PVI) to treat atrial fibrillation, a physician ablates tissue in a specific anatomical region, (e.g., an ostium of a PV). In order to verify that the region has been successfully isolated in PVI, the physician looks for conduction gaps in an ablation line caused by tissue that was not properly ablated. This is done by, among other techniques, visually identifying gaps between visual ablation tags that indicate the tissue locations where energy has been delivered.

When ablating in multiple locations and/or with a multi-electrode catheter, many ablation tags are created, and this produces visual clutter that causes difficulty for the physician to distinguish between visual ablation tags of different anatomical regions, making it harder to identify ablation gaps.

Moreover, due to the 3D visualization of the anatomy represented on a 2D display, ablation tags displayed in the background may get confused visually with tags of the region currently at the focus of the physician for ablation.

Some examples of the present disclosure that are described herein provide a technique comprising an algorithm and a graphical user interface (GUI) that allow users to categorize (e.g., set) anatomical regions, associate ablation visual tags to these regions and graphically encode each region with distinct graphics (e.g., color).

In some examples, the disclosed technique allows users to categorize, e.g., via the GUI, visual ablation tags quickly and easily to various categories which may represent different cardiac anatomical regions (e.g., areas) such as of a left superior pulmonary vein (LSPV), a right superior pulmonary vein (RIPV), and a cavotricuspid isthmus (CTI).

In other examples, the disclosed technique allows assigning visual ablation tags according to categories (e.g., according to anatomical regions or ablation stage) either prospectively or retrospectively.
∘ Prospectively means, for example, that before starting the ablation of a region (e.g. LSPV), the user sets a current tag region. All further ablation visual tags created will then be associated with that region and graphically encoded (e.g., colored) accordingly, until the user sets another tag region.
∘ Retrospectively means that a user can select and assign visual tags and/or ablation sessions to a previously created ablation region.

In an example, the disclosed technique provides a system (e.g., an EA mapping and ablation system) with the GUI comprising a predefined list of regions and associated shortcut keys for setting ablation visual tags to regions that may be defined by users.

In another example, the disclosed technique allows users to apply a different graphical encoding (e.g., color) for ablation visual tags which are sourced from any ablation energy type such as, but not limited to, radiofrequency (RF) or pulsed field (PF). The technique also allows users to graphically encode (e.g., color) ablation visual tags which are sourced from different ablation catheters, such as a tip catheter, a balloon catheter, and a basket catheter.

Selecting ablation visual tags may be done in a 3D map viewer by GUI tools, such as a "brush" tool that allows the user to click or drag the mouse cursor to select ablation tags directly from the map view.

The disclosed technique can have the processor apply/unapply (with GUI control or a shortcut key) a "Color by Region" color display, on the existing ablation visual tags, without losing existing information about the tag's original properties or original colors.

The disclosed technique allows a user to focus on specific region(s). For example, a physician may select a current area of interest for ablation by choosing (e.g. highlighting) one or more regions. Once the region or regions have been highlighted, all other ablation visual tags become semi-transparent or fully transparent, thereby allowing the user to focus visual attention only on the ablation tags within a desired region(s).

In yet another exmaple, the disclosed technique allows a processor to provide ablation result statistics within a graphically encoded region. Such statistics may include details about total net and gross ablation time within the encoded region, as well as the minimum and maximum distances between ablation tags within the region. Making such statistical information available per region may be beneficial for a study report, which currently must be calculated manually.

### SYSTEM DESCRIPTION

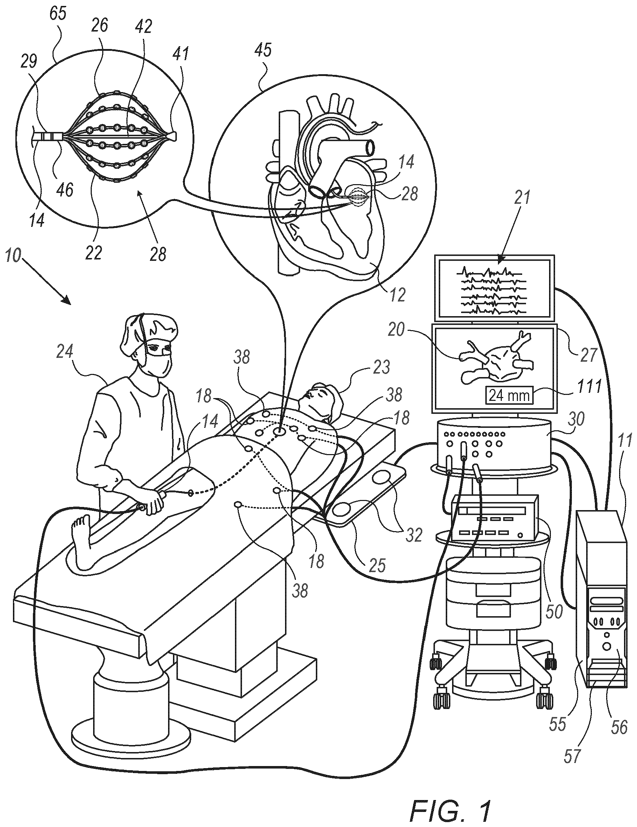

Fig. 1 is a schematic, pictorial illustration of a catheter-based electroanatomical (EA) mapping and ablation system 10, in accordance with an example of the present disclosure.

System 10 includes multiple catheters which are percutaneously inserted by physician 24 through the patient's vascular system into a chamber or vascular structure of a heart 12 (seen in inset 45). Typically, a delivery sheath catheter is inserted into a cardiac chamber, such as the left or right atrium near a desired location in heart 12. Thereafter, a plurality of catheters can be inserted into the delivery sheath catheter so as to arrive at the desired location. The plurality of catheters may include a catheter dedicated to pacing, a catheter for sensing intracardiac electrogram signals, a catheter dedicated to ablating and/or a catheter dedicated to both EA mapping and ablating. An example catheter 14, illustrated herein, is configured for sensing bipolar electrograms. Physician 24 brings a distal tip 28 (also called hereinafter distal end assembly 28) of catheter 14 into contact with the heart wall for sensing a target site in heart 12. For ablation, physician 24 similarly brings a distal end of an ablation catheter to a target site.

As seen in inset 65, catheter 14 is an exemplary catheter that includes a basket distal end 28, including one, and preferably multiple, electrodes 26 optionally distributed over a plurality of splines 22 at distal tip 28 and configured to sense IEGM signals. Catheter 14 may additionally include a position sensor 29, embedded in or near distal tip 28 on a shaft 46 of catheter 14, to track the position and orientation of distal tip 28. Optionally, and preferably, position sensor 29 is a magnetic-based position sensor including three magnetic coils for sensing three-dimensional (3D) position and orientation. As seen, distal tip 28 further includes an expansion/collapse rod 42 of expandable assembly 28 that is mechanically connected to basket assembly 28 at a distal edge 41 of assembly 28.

The magnetic-based position sensor 29 may be operated together with a location pad 25 that includes a plurality of magnetic coils 32 configured to generate magnetic fields in a predefined working volume. The real-time position of distal tip 28 of catheter 14 may be tracked based on magnetic fields generated with location pad 25 and sensed by magnetic-based position sensor 29. Details of the magnetic-based position sensing technology are described in U.S. Patent Nos. 5,5391,199; 5,443,489; 5,558,091; 6,172,499; 6,239,724; 6,332,089; 6,484,118; 6,618,612; 6,690,963; 6,788,967; 6,892,091.

System 10 includes one or more electrode patches 38 positioned for skin contact on patient 23 to establish a location reference for location pad 25 as well as impedance-based tracking of electrodes 26. For impedance-based tracking, electrical current is directed toward electrodes 26 and sensed at electrode skin patches 38 so that the location of each electrode can be triangulated via electrode patches 38. Details of the impedance-based location tracking technology are described in US Patent Nos. 7,536,218; 7,756,576; 7,848,787; 7,869,865; and 8,456,182.

A recorder 11 displays cardiac signals 21 (e.g., electrograms acquired at respectively tracked cardiac tissue positions) acquired with body surface ECG electrodes 18 and intracardiac electrograms acquired with electrodes 26 of catheter 14. Recorder 11 may include pacing capability to pace the heart rhythm, and/or may be electrically connected to a standalone pacer.

System 10 may include an ablation energy generator 50 that is adapted to conduct ablative energy to one or more electrodes at a distal tip of a catheter configured for ablation. The energy produced by ablation energy generator 50 may include, but is not limited to, radiofrequency (RF) energy or pulsed-field ablation (PFA) energy, including monopolar or bipolar high-voltage DC pulses, to be used to effect irreversible electroporation (IRE), or combinations thereof.

The patient interface unit (PIU) 30 is an interface configured to establish electrical communication between catheters, electrophysiological equipment, power supply, and a workstation 55 to control system 10 operation and receive EA signals from the catheter. Electrophysiological equipment of system 10 may include, for example, multiple catheters, location pad 25, body surface ECG electrodes 18, electrode patches 38, ablation energy generator 50, and recorder 11. Optionally, and preferably, PIU 30 additionally includes processing capability for implementing real-time computations of catheter locations and for performing ECG calculations.

Workstation 55 includes memory 57, a processor 56 unit with memory or storage with appropriate operating software loaded therein, and user interface capability. Workstation 55 may provide multiple functions, optionally including (i) modeling endocardial anatomy in three-dimensions (3D) and rendering the model or anatomical map 20 for display on a display device 27, (ii) displaying on display device 27 activation sequences (or other data) compiled from recorded cardiac signals 21 in representative visual indicia or imagery superimposed on the rendered anatomical map 20, (iii) displaying real-time location and orientation of multiple catheters within the heart chamber, and (iv) displaying sites of interest on display device 27 such as places where ablation energy has been applied. One commercial product embodying elements of system 10 is available as the CARTO^{™} 3 System, available from Biosense Webster, Inc., 31A Technology Drive, Irvine, CA 92618.

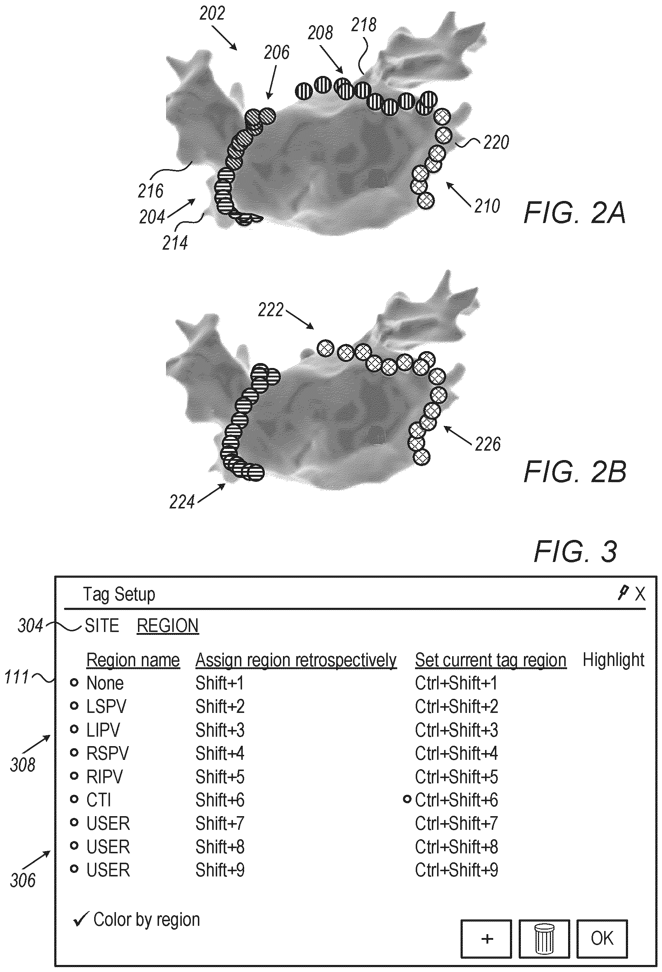

In the disclosed example, processor 56 runs an algorithm that allows physician 24 to set anatomical regions, associate ablation visual tags to these regions and graphically encode (e.g., color) each region with distinct graphics (e.g., color). In some examples, the disclosed technique allows users to quickly and easily categorize, e.g., via a GUI 111, ablation visual tags to various categories which may represent different cardiac anatomical regions (e.g., areas), as shown in Figs 2-4.

In some examples, processor 56 typically comprises a general-purpose computer, which is programmed in software to carry out the functions described herein. The software may be downloaded to the computer in electronic form, over a network, for example, or it may, alternatively or additionally, be provided and/or stored on non-transitory tangible media, such as magnetic, optical, or electronic memory.

This configuration of system 10 is shown by way of example, in order to illustrate certain problems that are addressed by examples of the present disclosure and to demonstrate the application of these examples in enhancing the performance of such a system. Examples of the present disclosure, however, are by no means limited to this specific sort of example system, and the principles described herein may similarly be applied to other sorts of medical systems. For example, other multi-electrode catheter types may be used, such as the OCTARAY^{™} catheter or a flat catheter.

### GRAPHICAL ENCODING OF ABLATION SITES ACCORDING TO

### CATEGORY

As noted above, the disclosed technique allows for graphically encoding ablation sites on an EA map according to their respective categories. The graphical encoding can be done manually by using a GUI, or automatically by using an encoding algorithm.

Figs. 2A and 2B are EA maps 202 and 222 superimposed with ablation sites graphically encoded (204, 206, 208, 210) according to anatomical regions and graphically encoded (224, 226) according to types of ablation sources, respectively, in accordance with an example of the present disclosure.

Fig. 2A shows the four PV ostia regions (214, 216, 218, 220) of a left atrium that requires PVI to isolate arrhythmia. As seen, some of the regions form a continuous region. The disclosed graphical encoding technique allows the physician to more easily identify conduction gaps in the ablation line that affect a particular ostium.

Fig. 2B allows the physician to differentiate ablation tags belonging to RF ablation (224) from those belonging to PF ablation (226). Based on the graphically encoded information, the physician can estimate the results of ablation, and, if needed, apply corrections at particular regions. In Fig. 2B these regions are divided into right and left PV ostia.

As RF and PF have different impacts on tissue, including side effects (such as potential damage to the esophagus), different regions may be optimally treated by a particular ablation method. The disclosed graphical encoding can improve decision making by the physician and thereby improve safety and efficacy of the medical procedure.

Fig. 3 is a schematic illustration of a graphical user interface (GUI) 111 used for categorizing and graphically encoding an EA map according to anatomical regions and user-specified categories, in accordance with an example of the present disclosure. The example GUI 111 comprises site and region tabs 304. The shown GUI is for the left atrium and includes a list 308 of left atrium regions. GUI 111 further includes a list 306 of user-defined regions. In GUI 111, graphical encoding of ablation sites and regions is performed by assigning them different colorings.

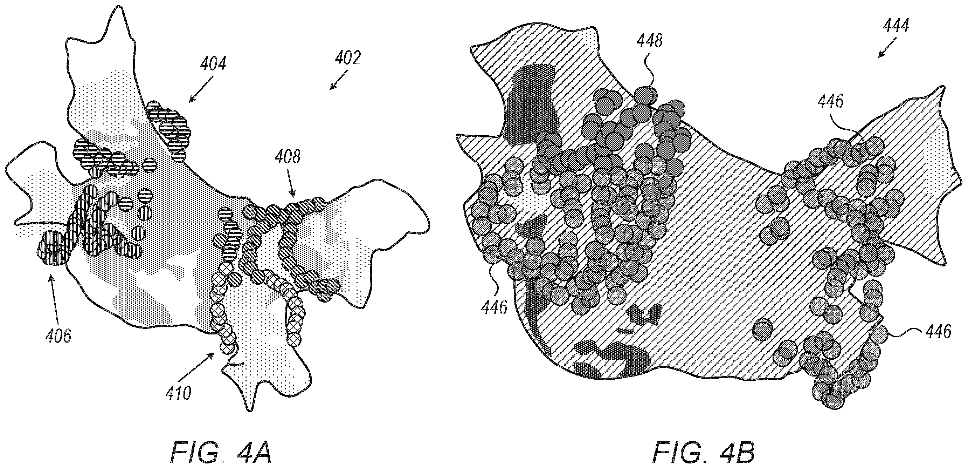

Figs. 4A and 4B are EA maps 402 and 444 superimposed with graphically encoded ablation sites (404, 406, 408, 410) according to anatomical regions (e.g., PV ostia), and (4B) with ablation sites graphically encoded (446, 448) according to user focus of interest, respectively, in accordance with an example of the present disclosure.

The graphical encoding in Fig. 4A increases the physician's awareness of both the anatomical regions and the planned and/or previously performed ablations. In Fig. 4A the physician graphically encoded anatomical regions and associated ablation visual tags (404, 406, 408, 410) to these regions by coloring sites in each region with distinct graphics (e.g., color), as well as coloring the ablation sites.

The graphical encoding in Fig. 4B allows the physician to focus on the current area of interest for ablation, and to choose (e.g., to highlight) one or more regions upon which to focus. All other ablation visual tags 446 of the other regions then change to semi-transparent or fully transparent. Thus, the user will be able to focus visual attention only on the ablation tags 448 within a desired region.

### METHOD OF GRAPHICAL ENCODING OF ABLATION SITES ACCORDING TO CATEGORY

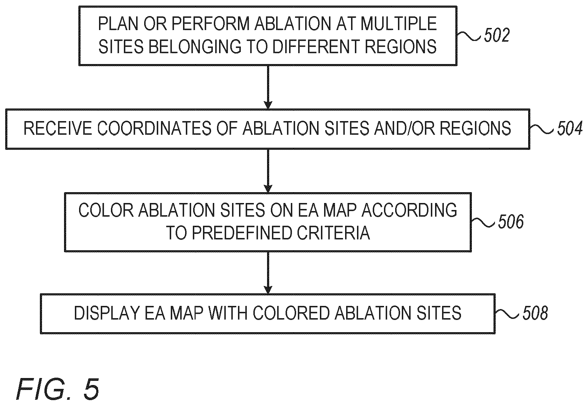

Fig. 5 is a flow chart that schematically illustrates a method to graphically encode ablation sites and regions on an EA map, in accordance with an example of the present disclosure. The algorithm, according to the presented example, carries out a process that begins with using system 10 to plan ablation or perform ablation at multiple sites belonging to different regions of an organ, such as a left atrium, at an ablation workflow step 502.

Next, the processor of the system, or another processor (e.g., of a workstation), receives coordinates of ablation sites and/or regions at ablation data receiving step 504.

At a coloring step 506, the processor graphically encodes the ablation sites on an EA map according to predefined criteria, as selected by a user with GUI 111.

Finally, at presenting step 508, the processor presents to a user the resulting EA map superimposed with the graphically encoded (e.g., colored) ablation sites, e.g., as seen in Fig. 4A.

The flow chart shown in Fig. 5 is chosen purely for the sake of conceptual clarity. Other examples of the technique may comprise different algorithmic steps, such as these that yield encoding seen in Figs. 2A, 2B and 4B.

### EXAMPLES

### Example 1

A method includes receiving (30) at least one of locations of multiple ablation sites and coordinates of one or more anatomical regions (214, 216, 218, 220) over a surface of a heart (12). The at least one of ablation sites and regions are categorized (304, 306, 308) according to predefined categories. The at least one of sites and anatomical regions are graphically encoded (204, 206, 208, 210, 224, 226, 404, 406, 408, 410, 446, 448) on an anatomical map (202, 222, 402, 444) according to their respective categories. The graphically encoded anatomical map (202, 222, 402, 444) is displayed (27) to a user.

### Example 2

The method according to example 1, wherein receiving (30) locations of multiple ablation sites comprises receiving at least one of planned and formed ablation sites.

### Example 3

The method according to any of examples 1 and 2, wherein the categories (304, 306, 308) comprise at least one of anatomical region (214, 216, 218, 220), type of ablation source (224, 226), and type of ablation catheter.

### Example 4

The method according to any of examples 1 through 3, wherein graphically encoding (204, 206, 208, 210, 224, 226, 404, 406, 408, 410, 446, 448) comprises at least one of coloring and texturing.

### Example 5

The method according to any of examples 1 through 3, wherein graphically encoding (204, 206, 208, 210, 224, 226, 404, 406, 408, 410, 446, 448) sites comprises tagging sites.

### Example 6

The method according to any of examples 1 through 3, wherein graphically encoding (204, 206, 208, 210, 224, 226, 404, 406, 408, 410, 446, 448) comprises using a graphical user interface (GUI) (111).

### Example 7

The method according to any of examples 1 through 6, wherein graphically encoding (204, 206, 208, 210, 224, 226, 404, 406, 408, 410, 446, 448) comprises using an automated algorithm.

### Example 8

The method according to any of examples 1 through 7, wherein graphically encoding (204, 206, 208, 210, 224, 226, 404, 406, 408, 410, 446, 448) comprises changing visibility of at least one of encoded sites and region between opaque, semi-transparent and fully transparent.

### Example 9

The method according to any of examples 1 through 8, wherein the anatomical map (202, 222, 402, 444) is an electroanatomical (EA) map.

### Example 10

The method according to any of examples 1 through 9, and comprising providing statistics of ablation results within at least one of the graphically encoded (204, 206, 208, 210) regions (214, 216, 218, 220).

### Example 11

A system (10) includes an interface (30) and a processor(56). The interface (30) is configured to receive at least one of locations of multiple ablation sites and coordinates of one or more anatomical regions (214, 216, 218, 220) over a surface of a heart (12). The processor (56) is configured to (i) categorize (304, 306, 308) the at least one of ablation sites and regions according to predefined categories, (ii) graphically encode (204, 206, 208, 210, 224, 226, 404, 406, 408, 410, 446, 448) the at least one of sites and anatomical regions on an anatomical map (202, 222, 402, 444) according to their respective categories, and (iii) display the graphically encoded anatomical map (202, 222, 402, 444) to a user.

Although the examples described herein mainly address cardiac diagnostic applications, the methods and systems described herein can also be used in other medical applications.

It will be appreciated that the examples described above are cited by way of example, and that the present disclosure is not limited to what has been particularly shown and described hereinabove. Rather, the scope of the present disclosure includes both combinations and subcombinations of the various features described hereinabove, as well as variations and modifications thereof which would occur to persons skilled in the art upon reading the foregoing description and which are not disclosed in the prior art.

## Claims

1. A method, comprising:
receiving (30) at least one of locations of multiple ablation sites and coordinates of one or more anatomical regions (214, 216, 218, 220) over a surface of a heart (12) ;
categorizing (304, 306, 308) the at least one of ablation sites and regions according to predefined categories;
graphically encoding (204, 206, 208, 210, 224, 226, 404, 406, 408, 410, 446, 448) the at least one of sites and anatomical regions on an anatomical map (202, 222, 402, 444) according to their respective categories; and
displaying (27) the graphically encoded anatomical map (202, 222, 402, 444) to a user.

2. The method according to claim 1, wherein receiving (30) locations of multiple ablation sites comprises receiving at least one of planned and formed ablation sites.

3. The method according to claim 1 or claim 2, wherein the categories (304, 306, 308) comprise at least one of anatomical region (214, 216, 218, 220), type of ablation source (224, 226), and type of ablation catheter.

4. The method according to any preceding claim, wherein graphically encoding (204, 206, 208, 210, 224, 226, 404, 406, 408, 410, 446, 448) comprises at least one of coloring and texturing.

5. The method according to any of claims 1 to 3, wherein graphically encoding (204, 206, 208, 210, 224, 226, 404, 406, 408, 410, 446, 448) sites comprises tagging sites, or using a graphical user interface (GUI) (111).

6. The method according to any preceding claim, wherein graphically encoding (204, 206, 208, 210, 224, 226, 404, 406, 408, 410, 446, 448) comprises using an automated algorithm, and/or changing visibility of at least one of encoded sites and region between opaque, semi-transparent and fully transparent.

7. A system (10), comprising:
an interface (30) configured to receive at least one of locations of multiple ablation sites and coordinates of one or more anatomical regions (214, 216, 218, 220) over a surface of a heart (12); and
a processor (56), which is configured to:
categorize (304, 306, 308) the at least one of ablation sites and regions according to predefined categories;
graphically encode (204, 206, 208, 210, 224, 226, 404, 406, 408, 410, 446, 448) the at least one of sites and anatomical regions on an anatomical map (202, 222, 402, 444) according to their respective categories; and
display the graphically encoded anatomical map (202, 222, 402, 444) to a user.

8. The system (10) according to claim 7, wherein the processor (56) is configured to receive locations of multiple ablation sites by receiving at least one of planned and formed ablation sites.

9. The system (10) according to claim 7 or claim 8, wherein the categories comprise at least one of anatomical region (214, 216, 218, 220), type of ablation source, and type of ablation catheter.

10. The system (10) according to any of claims 7 to 9, wherein the processor is configured to graphically encode (204, 206, 208, 210, 224, 226, 404, 406, 408, 410, 446, 448) by at least one of coloring and texturing.

11. The system according to any of claims 7 to 10, wherein the processor (56) is configured to graphically encode (204, 206, 208, 210, 224, 226, 404, 406, 408, 410, 446, 448) sites by tagging sites, or by using a graphical user interface (GUI) (111).

12. The system according to any of claims 7 to 11, wherein the processor (56) is configured to graphically encode (204, 206, 208, 210, 224, 226, 404, 406, 408, 410, 446, 448) by using an automated algorithm, and/or by changing visibility of at least one of encoded sites and region between opaque, semi-transparent and fully transparent.

13. The method according to any of claims 1 to 6 or the system according to any of claims 7 to 12, wherein the anatomical map (202, 222, 402, 444) is an electroanatomical (EA) map.

14. The system according to any of claims 7 to 13, the processor (56) is further configured to provide statistics of ablation results within at least one of the graphically encoded (204, 206, 208, 210, 224, 226, 404, 406, 408, 410, 446, 448) regions.

15. The method according to any of claims 1 to 6 and 13, and comprising providing statistics of ablation results within at least one of the graphically encoded (204, 206, 208, 210) regions (214, 216, 218, 220).
